# EUROPEAN PATENT SPECIFICATION

(11) **EP 2 836 171 B1**
(45) Date of publication and mention of the grant of the patent: **17.10.2018**
(21) Application number: 13775978.3
(22) Date of filing: 12.04.2013
(51) Int. Cl.: A61F 2/82, A61M 25/01, A61F 2/24

(54) **PERCUTANEOUS HEART VALVE DELIVERY SYSTEMS**
PERKUTANE HERZKLAPPENFREISETZUNGSSYSTEME
SYSTÈMES DE MISE EN PLACE PERCUTANÉE DE VALVULE CARDIAQUE

(30) Priority: 12.04.2012 US 201261623410 P; 13.08.2012 US 201261682663 P; 30.11.2012 US 201261732117 P
(43) Date of publication of application: 18.02.2015
(73) Proprietor: California Institute of Technology, Pasadena, CA 91125 (US); The Regents of the University of California, Oakland, CA 94607-5200 (US)
(72) Inventor: KHERADVAR, Arash, Irvine, California 92617 (US); KELLEY, Gregory, Santee, California 92071 (US); GHARIB, Morteza, Altadena, California 91001 (US)
(74) Representative: Murgitroyd & Company
(86) International application number: PCT/US2013/036469
(87) International publication number: WO 2013/155474

(56) References cited:
- WO-A1-2009/094189
- WO-A2-2005/107650
- WO-A2-2007/058847
- WO-A2-2008/029296
- US-A1- 2005 154 442
- US-A1- 2009 030 497
- US-A1- 2009 192 585
- US-A1- 2011 112 622
- US-A1- 2011 196 472
- US-A1- 2011 251 664

## Description

### RELATED APPLICATIONS

This filing claims the benefit of and priority to US Provisional Patent Application No. 61/732,117 filed November 30, 2012, US Provisional Patent Application No. 61/682,663 filed August 13, 2012, and US Provisional Patent Application No. 61/623,410 filed April 12, 2012.

### FIELD

The embodiments described herein relate to percutaneously-delivered heart valves and associated delivery systems.

### BACKGROUND

Transcatheter aortic valve replacement (TAVR) procedures require image- guidance during implantation to successfully deploy the heart valve into the correct position within the patient's aortic annulus. Current image technology uses X-Ray, CT, MRI, or ultrasound to visualize the surrounding anatomy. However, only X-Ray can be used during the procedure for image guidance. X-Ray is not sufficient for visualization because it is a 2D projection of 3D anatomy that depends on the orientation angle of visualization. Currently, other imaging modalities can be used prior to the procedure and during follow-up, with the hopes that anatomical visualization can be directly correlated to the X-Ray images seen during the procedure. However, differences in contrast, resolution, and artifacts can produce differing results.

Correct valve positioning is crucial for treatment success and optimal outcomes after transcatheter valve implantation. For example, to maintain a stable and correct lengthwise position with respect to the aortic annulus, a stepwise deployment that allows the valve to be repositioned both circumferentially and in the axial direction (i.e., towards the left ventricle (LV) or the ascending aorta) is important.

However, most of the current technologies are limited by instant deployment, and once the valve is deployed, repositioning and/or percutaneous retrieval is not possible - or at least difficult or potentially problematic. Placement of the stented valve in a position that is too high (or proximal) can totally or partially obstruct the coronary ostia in a case of aortic implantation, which may result in myocardial infarction or ischemia. Additionally, if the valve is placed too high in the aorta, it may embolize into the aorta causing significant paravalvular regurgitation. On the other hand, implantation in a position that is too low (or distal) is accompanied by compression of the atrioventricular (AV) node in the membranous septum, which leads to conduction abnormalities.

Further technical developments with a focus on a positionable, repositionable, and/or percutaneously retrievable valve design allow optimal placement and may thereby significantly reduce the risk of paravalvular aortic regurgitation, myocardial infarction, or ischemia related to improper positioning. Likewise, advances in imaging to facilitate optimal heart valve placement are needed.

WO2007/058847 A2 discloses a medical implant deployment tool and method and a deployment tool adapted to deploy the implant, the deployment tool having an actuation controller and plurality of actuation elements adapted to apply forces to one or more implant deployment mechanisms and each adapted to extend along an actuation element path within a patient's vasculature; and an actuation element compensation mechanism adapted to compensate for differences in length between the actuation element paths.
WO 2009/094189 A1 discloses a delivery system for delivering a stented prosthetic heart valve to a lumen of a patient, the delivery system including a tubular body having a proximal end and a distal end, and a plurality of wires extending from the distal end of the tubular body, wherein each of the wires has a distal end that is coiled for engagement with a stent of a stented prosthetic heart valve and a method of deploying an implantable stented device using such a delivery system comprising axially moving at least one of the plurality of wires relative to the tubular body to disengage the coiled distal end of the wire from the stent engagement component with which it is engaged.

### SUMMARY

The embodiments described herein address the need for improved catheter devices for coordinated delivery, positioning, repositioning and/or percutaneous retrieval of the percutaneously implanted heart valves. The delivery system apparatus is a tool that may incorporate a guide wire lumen. As such, a given device may be suitable for so- called "over-the-wire" use and include a delivery sheath covering that restrains the stent frame of the valve. Alternatively, the delivery device may be tracked trough a catheter serving such function, as in a so-called "guide" or "delivery" catheter.

In one embodiment, the delivery apparatus includes a number of arms (such as, but not limited to three) embedded within its body that hold the valve's stent during the delivery procedure when it is in the collapsed state. The arms are equipped with adjustable springs that are remotely controllable by the operator, and allow for robust radial expansion or deployment of the collapsed stent in increments.

In use, the arms remain attached to the valve stent frame until the stent frame is fully deployed. If the stent/stent frame is not properly deployed, the arms, which are still releasably attached to the stent until intended release, can be used for partial contraction of the stent for repositioning purposes. When the stented valve is properly positioned as desired within the heart, the arms will be released from the stent, and return to their embedded/retracted positions within the apparatus. Then the entire apparatus is retracted. It may be retracted from the heart or vasculature over any guide wire used and/or through any delivery catheter employed for site access.

In another system embodiment allowing for stented valve delivery, repositioning, and/or percutaneous retrieval, draw line filaments are positioned through the distal end of a pusher sleeve (or draw tube), along a lumen of the sleeve (or tube), out through holes in the sleeve (or tube), out through proximal frame holes, along the surface of a heart valve frame, in through distal frame holes, in through the distal end of the sleeve (or tube), along the lumen of the sleeve (or tube), and out the proximal end of the sleeve (or tube). Variations on this approach are possible as are various optional features of the stent frame facilitating such use.

The draw lines may comprise polyester (PE), PTFE, suture material, or another high strength (and preferably biocompatible fiber) braid or bundle of fibers such as ultrahigh-molecular-weight polyethylene (UHMWPE, sometimes shortened to UHMW). In this embodiment and others described herein, the heart valve frame may comprise superelastic NiTi alloy heatset in a desired shape, it may be constructed of a so-called "engineering plastic" such as polyetheretherketone (PEEK) or may be constructed otherwise. Various surface treatments or finishes may be desirable. In the case of a NiTi (Nitinol) or another metallic material implant, an electro-polished surface may be preferred.

Collapsed and expanded states of a heart valve can be controlled by varying the position and/or tension applied to the draw lines. A customized handle may be provided for user interface. Draw line tension can be increased until the heart valve frame is fully collapsed and fully releasing the draw line tension allows the self-expanding heart valve frame to fully expand. The heart valve frame may be put in an intermediate state by varying the tension applied to the draw lines. Moreover, the system can be setup to allow a range of lateral control of the stent position during delivery. In one variation, a "joystick" control interface is provided; in another a model of the implant (or at least the stent frame portion of the valve to be delivered) is used.

In yet another delivery system embodiment allowing for delivery, repositioning, and/or percutaneous retrieval, different means or entities are provided to control the state of device deployment (variably, from fully collapsed to fully expanded) of the proximal end of a self-expanding heart valve device. Such means or entities pertain to the use of multiple sleeve or sheath features (herein optimally referred to as "zip tube" parts or an assembly with "zip tube" sheaths or fingers) provided to mechanically change an angle between adjacent strut elements and thereby the proximity of the struts. In use, the zip tube sheaths (or fingers) collapse the heart valve frame by "zipping" the struts into closer proximity.

In this embodiment, the ends of a self-expanding heart valve frame are configured with a link feature. A self-expanding retainer is constructed and configured with diametrically collapsible retainer arms or fingers. A zip tube part or assembly with diametrically expandable/collapsible sheath fingers is configured in such a manner to allow the zip tube fingers to slide over the retainer fingers. The ends of the retainer fingers are configured with a clasp or link feature so as to mate to the heart valve frame clasp or link features.

The zip tube assembly may be partially advanced (distally) to trap the heart valve frame and retainer such that they will not unlink because the inner diameter (or inner dimension(s)) of the zip tube fingers are constructed so as to constrain the linked heart valve frame and retainer from unlinking when positioned around the linked frame or retainer. With the retainer serving as a means to secure the valve in position, the zip tube assembly may be variably advanced (relative to the linked heart valve frame or retainer) to variably (e.g., partially) collapse the proximal end of the heart valve device or fully advanced to fully collapse the proximal end of the heart valve device.

The zip tube part assembly may be variably retracted to allow the proximal end of the self-expanding heart valve device to variably (partially) expand or retracted sufficient to allow the self-expanding heart valve device to fully expand. Alternatively, the zip part or assembly may be secured in position and the retainer may be variably retracted to variably collapse the proximal end of the heart valve device up to fully collapsed or variably advanced to allow the self-expanding heart valve device to variably expand up to fully expanded. The zip tube part or assembly can be fully retracted allowing the heart valve frame and retainer to unlink thereby releasing the heart valve device from the delivery system so that the heart valve device may be left in position and the delivery system may be removed.

In addition, any of the subject delivery system architectures may incorporate a visualization system for image-directed heart valve delivery. Alternatively, other features for restraining and/or manipulating a self-expanding stent frame or a ballooned stent frame approach may be employed in an image-guided system. All of these embodiments involve a catheter or catheter-like device that utilizes an integrated imaging modality with a deployment mechanism. As such, these embodiments may be used to accurately deploy a heart valve into a patient with greater accuracy and precision than with current procedural imaging modalities where direct visual confirmation is not possible.

In these embodiments, the delivery system incorporates a catheter-based imaging modality within the device, such as, but not limited to, intravascular ultrasound (IVUS), intravascular photoacoustic (IVPA), optical coherence tomography (OCT), raman spectroscopy, or an optical method, capable of detecting features of a vessel in which the catheter is inserted. The selected imaging systems allow clinicians to image both the surrounding anatomy and the advancing catheter in real-time during the procedure.

In one example, since IVUS is a tomographic imaging modality, a 3D image of the aortic root can be produced through pull-back imaging. High-resolution IVUS is well-known for interrogating the lumen wall of vessels and has also been used to visualize metal stents in vivo. In the example of IVUS hardware, a physician can accurately image and position the implantable valve device without the use of ionizing radiation or nephrotoxic contrast agents. Furthermore, IVUS advantageously provides for a real-time imaging modality.

A catheter system can be based upon an imaging catheter or a valve delivery catheter. In an embodiment where the catheter system is based upon the valve delivery catheter, the imaging modality device can be inserted through the center of the valve delivery catheter, where the active imaging element is aligned with a feature of the valve delivery catheter, such as, but not limited to the catheter tip, the distal or proximal end of the valve stent, or some other pre-determined landmark of the valve delivery catheter. Positioning of the imaging device on the circumference of the valve delivery catheter is also possible in another embodiment to prevent visual hindrance from the implanted stent.

In yet another embodiment, the valve delivery system is based upon the imaging catheter, and the deployment mechanism is inserted through the lumen of the imaging catheter, such as, but not limited to, through a guidewire port of the imaging catheter. Furthermore, the delivery system referred herein is not limited to the delivery of a heart valve device, but could be used to deliver therapy to a localized region through the use of a catheter. Such examples of delivery could include, but are not limited to, delivery of drugs or other therapeutic agents, delivery of RF irradiation, or delivery of another device.

Operation of the delivery system allows visualization of the surrounding anatomy during insertion of the imaging catheter in the context of the location of the delivery catheter. As such, the location of the delivery catheter relative to the surrounding environment may always be known. In one embodiment, the delivery system is fixed relative to the imaging transducer within the catheter. In another embodiment, the two components can be moved relative to one another. However, in embodiments where relative motion is allowed, the relative motion is advantageously tracked or known in order to maintain accuracy in the advancing catheter.

Accordingly, the invention provides a medical device delivery system as defined in the appended claims.

The subject delivery devices, kits in which they are included (with and without valve installation or assembly), methods of use and manufacture (such as assembly of the delivery system and frame alone and/or with included valve) are all included within the scope of the present disclosure. Some aspects of the same are described above; more detailed discussion is presented in connection with the figures below.

Other systems, devices, methods, features, and/or advantages of the subject matter described herein will be or will become apparent to one with skill in the art upon examination of the following figures and detailed description. It is intended that all such additional systems, devices, methods, features, and/or advantages be included within this description and be within the scope of the subject matter described herein, regardless of whether recited in this summary section. In no way should the features of the example embodiments in this or any other section be construed as limiting the appended claims, absent express recitation of those features in the claims.

### BRIEF DESCRIPTION OF THE DRAWINGS

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely. Variations other than those shown in the figures are contemplated as described in a broader sense in the above summary section, as generically claimed, or otherwise.
Figs. 1A-1F are perspective views illustrating an example embodiment of a stent frame and valve in various stages of deployment as may be employed in connection with the embodiments herein.
Fig. 2A is a detail view illustrating the delivery device sleeve of a first embodiment showing the location of one of a plurality of embedded arms.
Fig. 2B is a detail view illustrating the arm at the location in Fig. 2A connected to a spring system for controlling stent frame deployment.
Fig. 3 is a system overview illustrating the arms releasably attached to a stent frame.
Fig. 4A is a detail view illustrating the arms fully extended from the delivery apparatus and Fig. 4B is a detail view illustrating a hollow deployment arm with strings inside and a pull/push mechanism inside the guide tube or sleeve.
Figs. 5A-5E illustrate progressive stages of stent frame deployment and recapture for a second embodiment.
Figs. 6A-6C illustrate side, end, and perspective views, respectively, of the delivery device sleeve of the second embodiment.
Figs. 7A and 7B are side views illustrating the sent frame associated with the delivery device sleeve in contracted and expanded states, respectively.
Fig. 8A illustrates a variation of the subject stent frame and Fig. 8B illustrates a variation of the subject delivery sleeve with associated draw line filaments.
Figs. 9A-9C are side, perspective, and end views, respectively, illustrating the components in Figs. 8A and 8B assembled together.
Figs. 10A and 10B are side and end views, respectively, illustrating the same assembled components shown in a compressed state.
Figs. 11A and 11B are partial perspective and detail side views, respectively, illustrating a stent frame for a third embodiment.
Fig. 12 is a perspective view illustrating a frame retainer with retainer fingers.
Figs 13A and 13B are perspective and end views, respectively, illustrating a zip tube part or assembly and zip tube fingers.
Fig. 14A illustrates segments of an expanded heart valve frame, retainer fingers, and zip tube fingers as associated in the subject embodiment and Fig. 14B illustrates a complete assembly of the embodiment including these subcomponents.
Figs. 15A-15F are detail side views illustrating operation of elements within the embodiment.
Figs. 16A and 16B are side views illustrating an example embodiment of imaging catheter and stent frame components of an imaged-guided delivery system.
Fig. 17 is an enlarged perspective view of a stent frame component as previously illustrated.
Figs. 18A and 18B are side views illustrating the stent frame embodiment of Fig. 17 associated with a delivery device, with the stent frame in a neutral and a laterally displaced position, respectively.
Figs. 19A and 19B are photographs illustrating prototype hardware of the delivery system embodiment diagrammatically illustrated in Figs. 18A and 18B.
Fig. 20 diagrammatically illustrates an alternative user interface for the Fig. 18A and 18B delivery system.

### DETAILED DESCRIPTION

Various example embodiments are described below. Reference is made to these examples in a non-limiting sense, as it should be noted that they are provided to illustrate more broadly applicable aspects of the devices, systems and methods as defined in the appended claims.

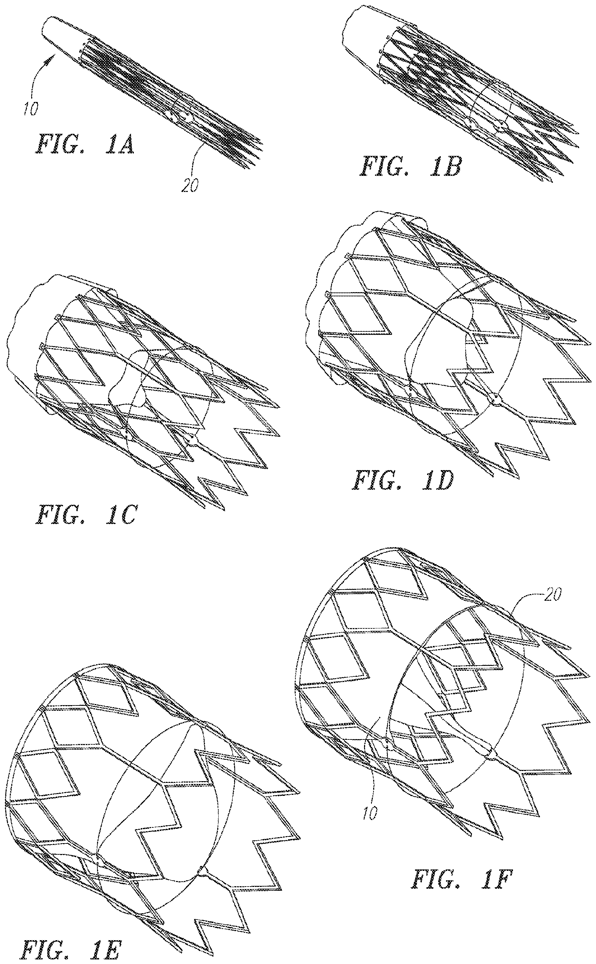

Figs. 1A-1F illustrate an implant 2 and a suitable approach to valve 10 attachment and its manipulation for delivery in coordinated use with an expandable stent frame 20. Further details as to valve construction and/or its manipulation for delivery may be appreciated in review of USPN 8,133,270 to Kheradvar, et al. Features of the stent frame elaborated upon below in the various embodiments may be added to those shown in Figs. 1A-1F or used in connection with other suitable stent frame and/or other valve architectures.

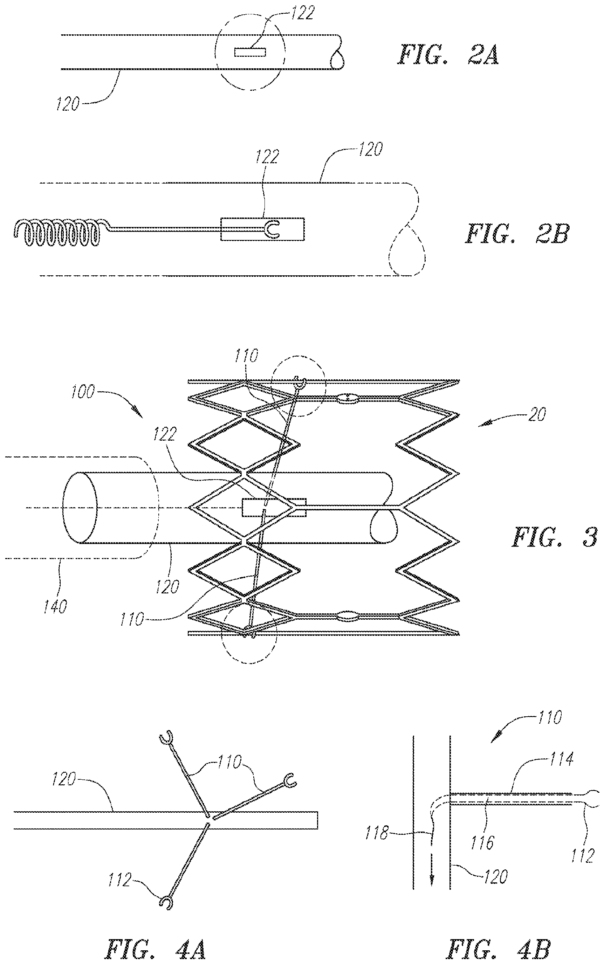

In any case, implant 2 (e.g., valve 10 and stent frame 20) is directly applicable for coordinated use with a delivery system as shown in Figs. 2A-4B. More specifically, a delivery system apparatus for controlled deployment of a stented heart valve system in increments is shown. The system provides for repositioning a stented heart valve system during and after deployment. As variously illustrated, device 100 includes a plurality of deployable arms 110. These are adjustably deployable. The arms are first embedded inside the apparatus. Fig. 2B illustrates the location of one of the embedded arms 110 within a delivery device sleeve 120. For tracking to the target site, the arms are hidden. The arms exit the sleeve through ports or slots 122 in the wall of the sleeve. The arm lengths are adjustable and the arms are releasably attached to the stent of the stented valve. As shown in Fig. 2B, each arm may be equipped with an in-line adjustable spring that is controllable by the operator remotely. As illustrated in Fig. 3, such actuation allows for robust radial expansion or deployment of the collapsed stent frame in increments.

The arms remain attached to the stent until the stent is fully deployed. During tracking to a site for deployment, the stented valve may be covered by a sheath incorporated in the delivery system or pass within a delivery catheter (either case illustrated by an optional sleeve 140). If the stent is not properly deployed, the arms, which are still releasably attached to the stent, can be used for partial contraction of the stent for repositioning purposes. When the stented valve is properly positioned within the heart, the arms will be released from the stent, and return to their embedded positions within the apparatus. Then the apparatus will be retracted into the sheath or through the delivery catheter from the heart or vasculature.

As seen in Fig. 4A in which the stent frame is detached, each arm may terminate in a releasable hook, jaw, clevis 112 or the like for such purpose(s). The connection and release may be provided by a simple snap fit. Otherwise it may be provided by a more active means for stent frame interface as illustrated in Fig. 4B, that shows an arm comprising a hollow micro tube or sheath 114 with spring loaded strings or filaments 116 inside where a string or filament 118 inside the guide tube or sleeve 120 can be used to control the closing and opening of the hooks 112.

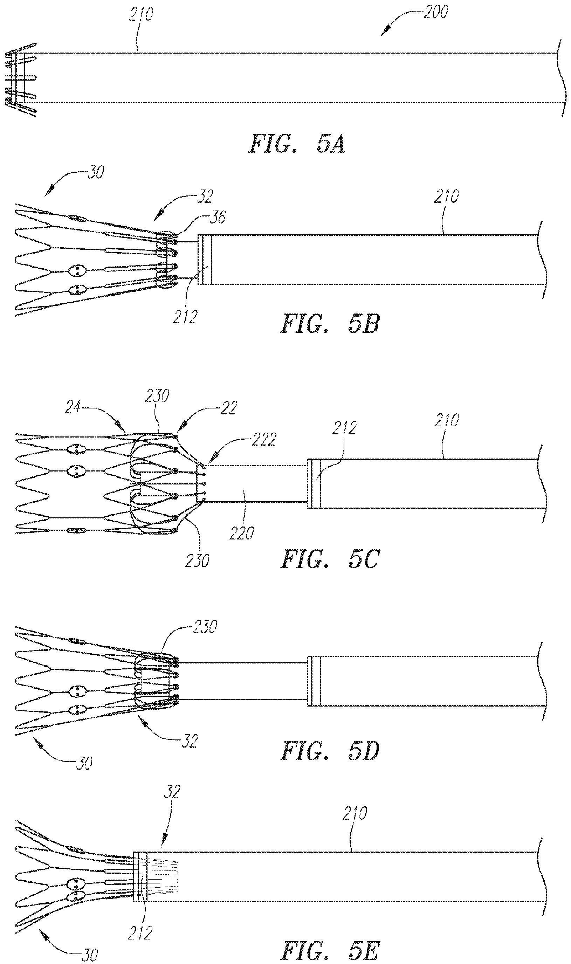

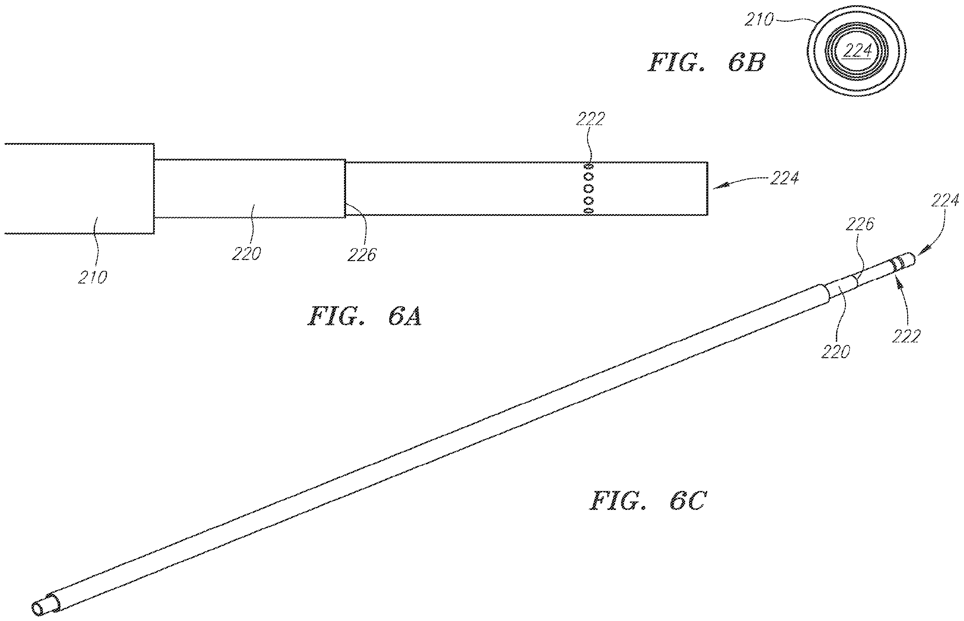

Figs. 5A-5E illustrate progressive stages of implant deployment and recapture for a second embodiment. Here, in a system pictured for over-the-wire tracking to its deployment site, a delivery system 200 includes a sheath 210 (with distal radiopaque marker 212) coaxial with a pusher sleeve 220. A distal portion of sleeve 220 includes apertures 222 through which filaments 230 pass into and proximally within the length of the sleeve. The filaments loop from these apertures through proximal stent frame apertures 22 and more distal stent frame apertures 24 (or alternatively past strut junctions in a different stent configuration) and into a distal end 224 of the sleeve (or a second set of distal apertures (not shown) in the sleeve if so-desired). Such details of the sleeve are shown unobscured in Figs 6A-6C, as is an optional shoulder 226 for abutting proximal end or crown sections 26 of the stent frame and guide sheath 210 of the proximal end or crowns of the stent frame.

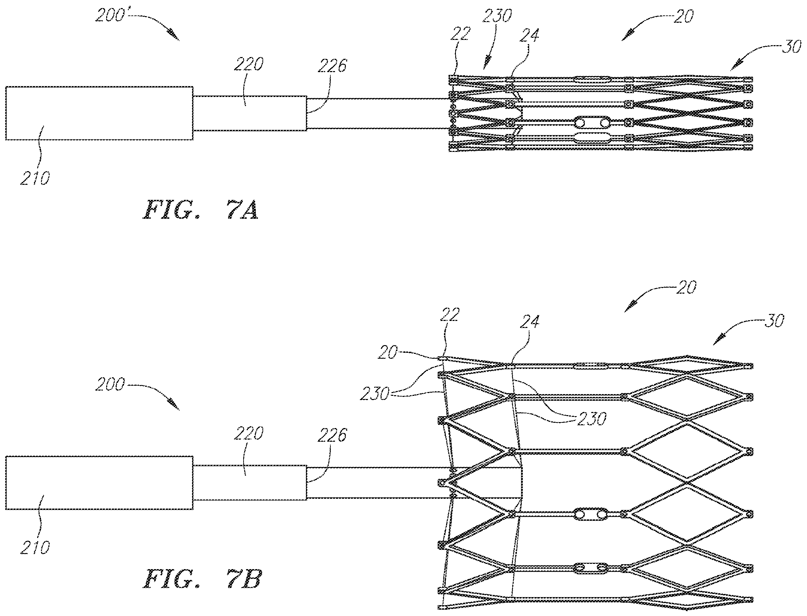

Regarding interaction between the stent frame and delivery system 200, Figs. 7A and 7B provide side views of the stent frame associated with the delivery device sleeve in contracted and expanded states, respectively. Here, the manner of stent frame expansion and contraction as related to extended filament 230 length is clearly visible.

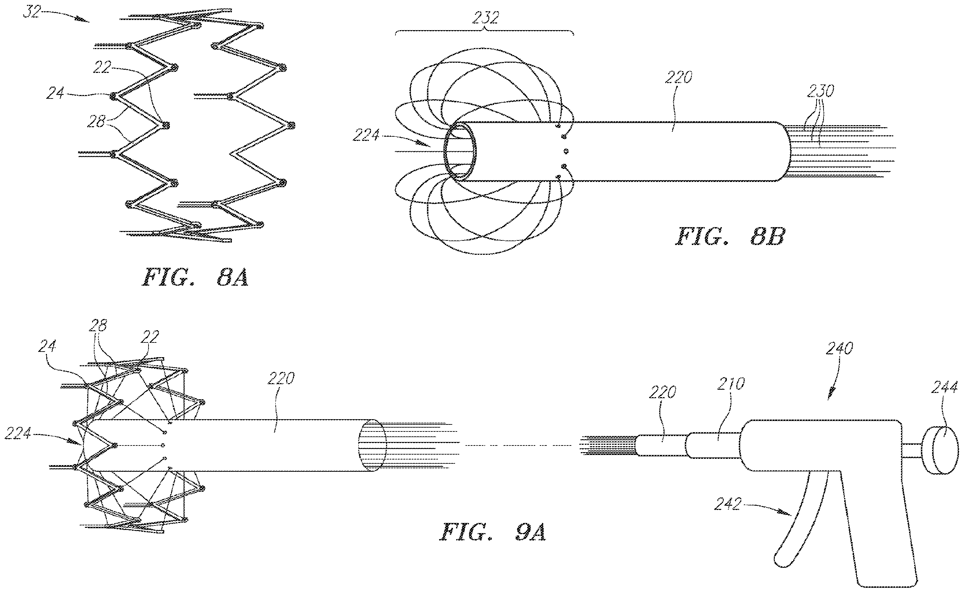

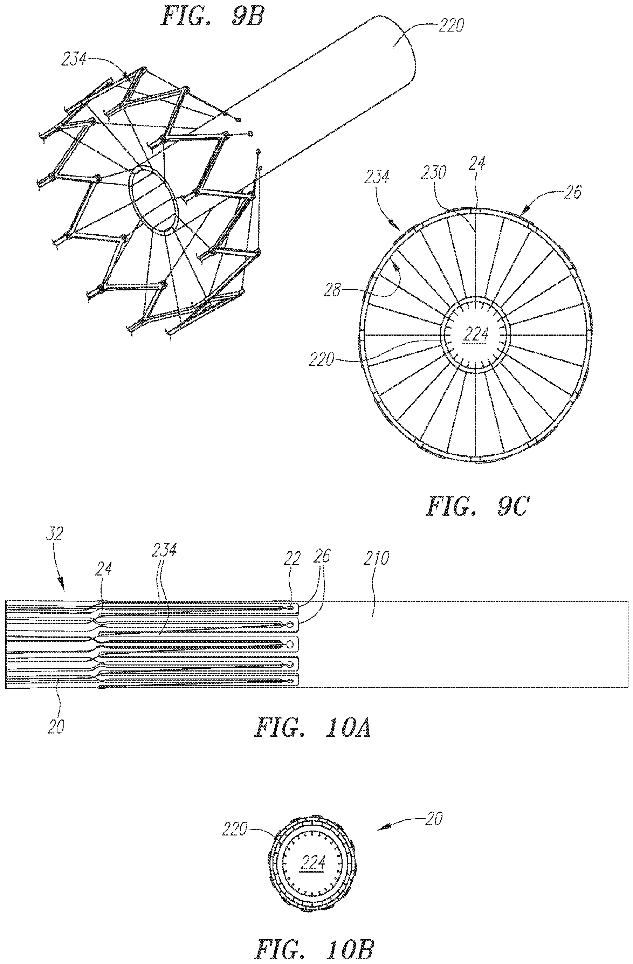

Figs. 8A and 8B further illustrate such details as described above. When assembled in a delivery system 200, stent frame 20 will be captured within loops 232. The assembled relation of elements is shown in each of Figs. 9A-9C and Figs. 10A and 10B. Comparing Figs. 9A-9C to Figs. 10A and 10B, the state of the stent frame is changed from open or expanded in the former trio of figures, to compressed in the latter pair.

Such control is achievable by remote actuation of the loop filaments with a customized handle or other user interface means. Any handle may include means for group control of the filaments and independent control of sheath position. Such a handle 240 may include separate "grip" 242 and "plunger" or "slide" 244 interfaces as illustrated by example in Fig. 9A for such purposes. Otherwise, mechanism internal to the handle can automate all of the various control procedure(s) by actuating a grip 242, alone.

Figs. 9A and 9B also offer good illustration of the manner in which filaments 230 pass through apertures 22, 24 and run along interposed strut sections 28. Fig. 9C illustrates the radial relationship of the apertures and filament 230 portions. Here, a crossing segment 234 of the filament between the apertures 22 and 24 is positioned outside of and opposing strut section 28. The crossing segments are angled with the struts when the stent frame is in an expanded state and more close to axially aligned when the stent is compressed as shown in Figs. 10A and 10B.

As noted above, the transition between the open and compressed states (and states therebetween) is managed by letting-out or reeling-in the draw line filament determining the size of the control loop. Ultimately, one end of the line is pulled all of the way through the stent aperture to finally release the implant.

Figs. 5A-5E illustrate a range of activity that is possible in terms of device manipulation before such release. In succession, these views show progressive stent frame deployment and steps toward complete recapture. Fig. 5A pictures (literally, given that the figures are based on photographs) the beginning of stent frame deployment as sheath 210 is withdrawn and a distal end 30 of the stent self-expands. Fig. 5B shows the sheath fully withdrawn and full tension on the draw lines or filaments, maintaining a proximal side 32 of the stent 20 in a compressed state. As in Fig. 5D illustrating the same (but in the case of Fig. 5D re-compression after the relaxation of draw lines to allow expansion as in Fig. 5C), the sheath can be advanced to fully recapture the stent frame. With the beginning of such action shown in Fig. 5E, the stent frame can be fully recovered within sheath 210 - whether for the purpose of repositioning or bulk retrieval of the device.

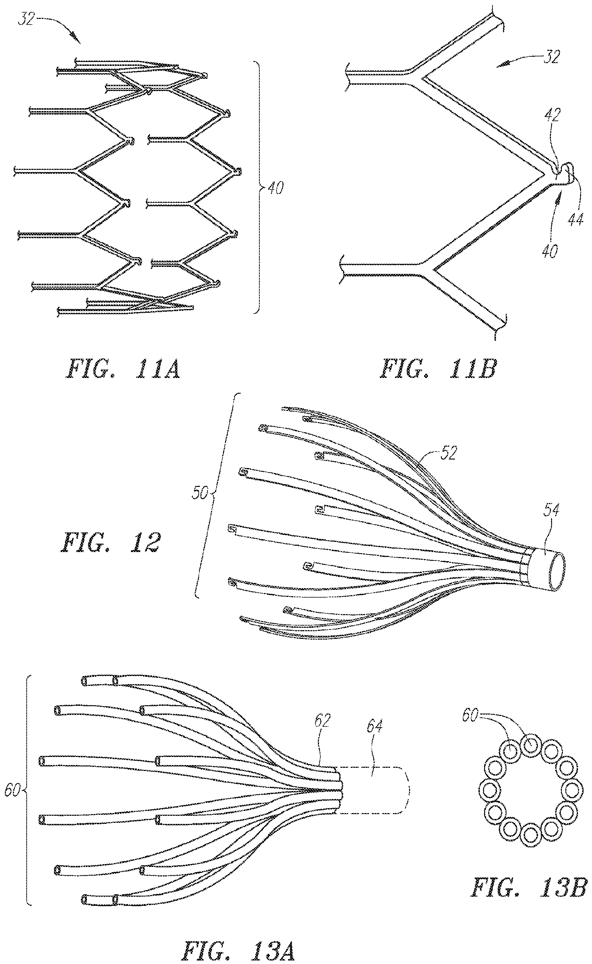

A third delivery device embodiment is able to offer similar advantages in terms of delivery, repositioning, and/or percutaneous retrieval. Stent frame components of such a system are shown in Figs. 11A and 11B. In each view, a proximal end 32 of a stent frame 20 includes clasp features 40. Each clasp feature 40 may comprise a bridge section 42 and an overhang section 44. Complementary clasp features 50 are provided at the end of resilient retainer "arms" or "fingers" 52 associated with a delivery system pusher. Arms 52 may comprise Nitinol or another elastic or superelastic material. Arms 52 are biased outward such that they spring out to a position as shown in Fig. 12 when released from restraint (e.g., upon exiting a delivery system sheath element or delivery/guide catheter body). Arms 52 are joined or meet at a hub 54. These components may be cut from a single hypotube or polymer sleeve that extends to the proximal end of the delivery system (not shown) as one piece or be assembled using conventional techniques such as laser welding, etc. In any case, pairs of complementary clasp elements 40/50 are releasably engaged in sheaths 60.

Figs. 13A and 13B illustrate a construct in which multiple sheaths 60 extend to and join at a hub 62 optionally extending proximally as a single sleeve 64. Such a structure can be produced by bundling and reconfiguring (e.g., by fusing) a plurality of thermoplastic sheaths, bundling and bonding a plurality of sheaths, and splitting an end of a multi-lumen extrusion into a plurality of separate sheaths. Other means of construction will be appreciated by those of skill in the art as well.

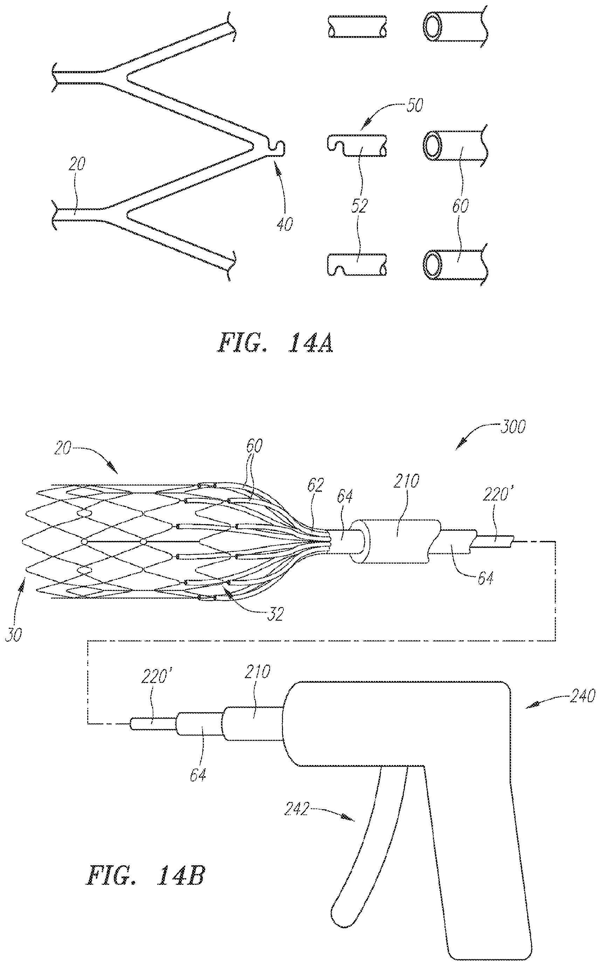

Regardless, Fig. 14A provides a partial assembly drawing illustrating the axial alignment for a plurality of interfacing members. Fig. 14B shows the same components of the third device embodiment brought together in a completed apparatus assembly 300. As in the embodiments above, such a system may optionally include a cover sheath 210 and a handle 240. In addition, system 300 may include an innermost elongate sleeve 220' optionally providing a lubricious PTFE liner for a guidewire lumen and/or column or "push" strength to the system.

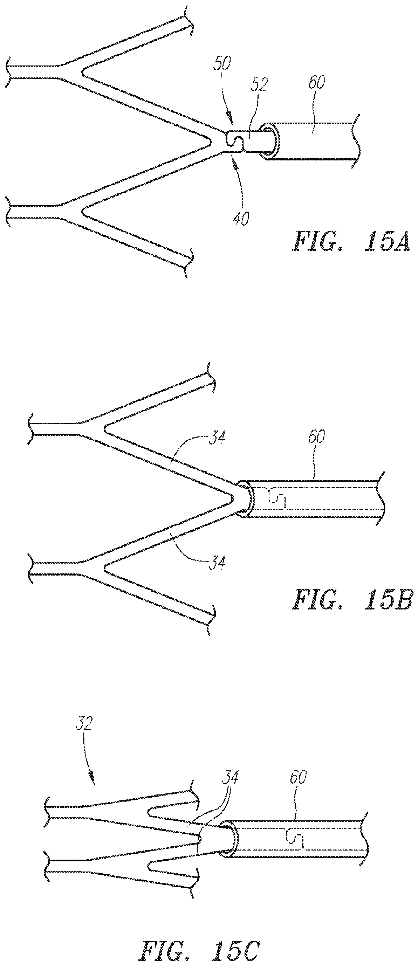

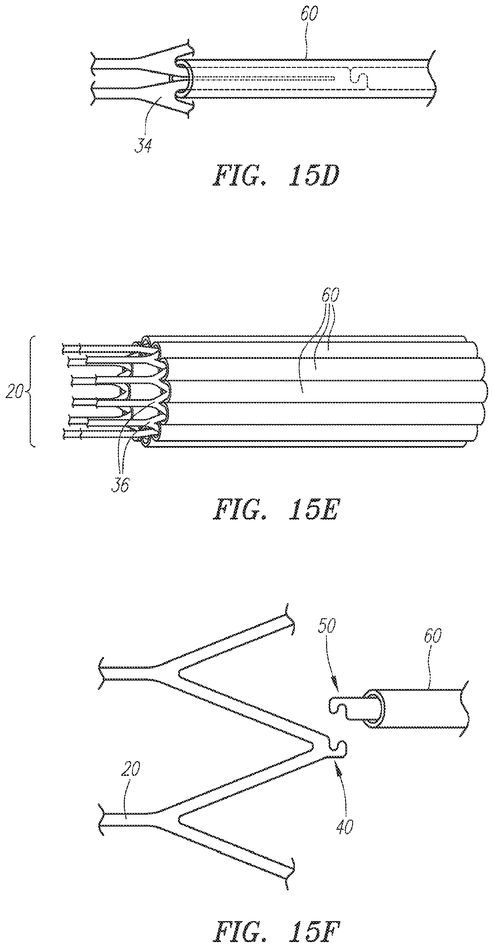

Figs. 15A-15F illustrate the operation of an intended interaction of the subcomponents of system 300. In Fig. 15A, the heart valve frame clasp or link 40 is interfaced with clasp/line 50. In Fig. 15B, clasps features 40/50 are trapped within sheath 60. At this point, further withdrawal of stent frame 20 into sheath element 60 or (stated otherwise) advancement of sheath 60 over adjacent proximal stent struts 34 results in a condition as shown in Fig. 15C. Here, struts 34 are brought together collapsing the entirety of the proximal end 32 of stent frame 20 (given that the same condition is achieved around the entire periphery of the stent by paired device features). As shown in Fig. 15D, sheath 60 can cover the entirety of struts 34 up to their junctions 36 with adjacent struts. The net effect is shown in Fig. 15E where the entire proximal side of the stent frame 20 is compressed efficiently by the multiple sheath elements shown.

As summarized above, the zip tub part assembly (sheaths 60 and associated components) may be variably retracted to allow the proximal end 32 of the stent frame to partially expand or retracted sufficiently to allow the stent frame to fully expand. Alternatively, the zip part/assembly may be secured in position and the arm retainer 54 retracted to variably collapse the proximal end of the heart valve device (up to fully collapsed) or variably advanced to allow the self-expanding heart valve device to variably expand (up to fully expanded). Further action associated with collapse/compression and expansion of the stent frame is achieved by covering and uncovering the stent frame with optional sheath 210 or by a guide catheter.

In any case, upon achieving desired implant placement, clasp elements 40/50 can be freed from confinement within the sheath member(s) 60 thereby unlinking the elements allowing stent frame 20 release as shown in Fig. 15F and allowing delivery system withdrawal from a patient in a successful percutaneous heart valve implantation procedure.

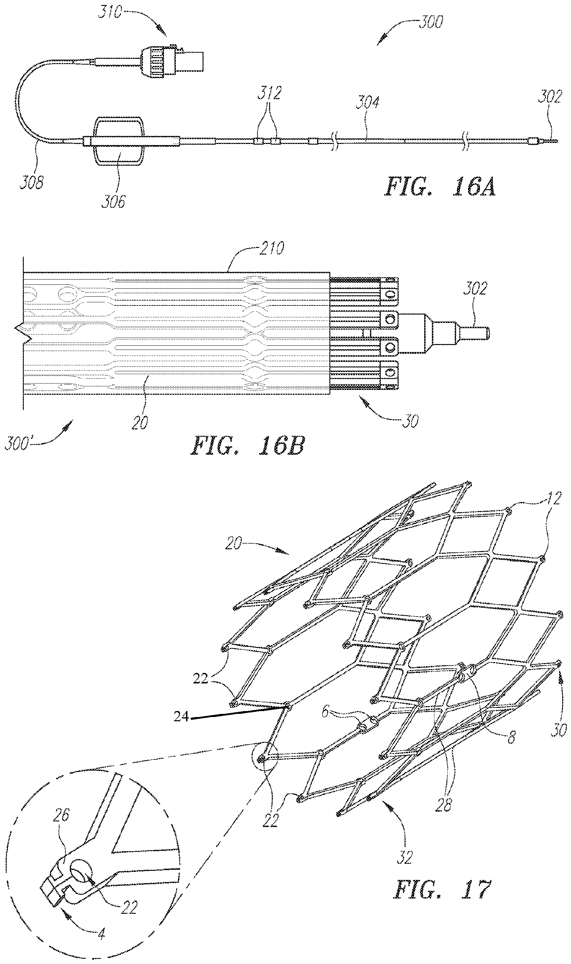

Fig. 16A illustrates a suitable IVUS catheter 300 for use in an image-guided valve delivery system according to another embodiment. The figure shows an EAGLE-EYE IVUS imaging catheter (Volcano Corp). Imaging catheter 300 includes a distal transducer tip 302, an intermediate catheter shaft or body 304, handle/grip 306, lead 308, and a proximal connector 310. Radiopaque shaft markers 312 are provided that may be relocated or additional markers added for coordination with a valve delivery catheter to (together) provide an overall valve delivery catheter system (e.g., by inserting catheter 300 within delivery system 100 or 200 as previously illustrated).

A distal portion of such a combined system 300' in shown in Fig. 16B. This photograph shows a distal end 30 of a TAVR stent 20 compressed to 4.3 mm diameter (13Fr). It is held in a sheath 210 that may form part of an overall delivery system 300'. Otherwise, it may be regarded as a loading sheath or surrogate (or stand-in) for a delivery catheter through which the stent 20 will track in a medical procedure. As shown, an ATLANTIS SR PRO IVUS transducer (Boston Scientific Corp.) 302 is placed through the center of the valve stent frame 20 for sizing purposes.

The image does not show the valve leaflets (e.g., as in Figs. 1A-1F) for the overall implant that contribute to the inner diameter space constraints or the specific delivery system features that may be employed. Yet, the image illustrates the general hardware (stent frame, delivery system/sheath components and IVUS device) that may be employed in the subject systems and methods.

Fig. 17 is a perspective view of a stent frame 20 component that may be employed therein. Actually, this figure provides an enlarged view of the stent frame shown in Figs. 7A and 7B. So-enlarged, features in addition to those of the stent in USPN 8,133,270 upon which the overall architecture may be based are easily highlighted. Specifically, two sets of holes 22 and 24 (proximal and more distal) are provided at the proximal side 32 of the stent frame 20 (i.e., on the "top" of the stent that would be positioned in the aortic root). These holes allow for passage of a network of pull-strings or filaments used for step-wise deployment, repositioning of the stent, and retrievability back to the guide-wire catheter (as discussed above) and also lateral positioning (as discussed below). Further, T-shaped structures 4 at the proximal side 32 are added to proximal crown features 26 to accommodate repositioning and retrievability of the valve during implantation procedure by way of attachment to complimentary delivery system features 40 like the example shown in Figs. 14A and 14B.

In addition, connector holes 6 in tabs 8 of material at the middle of a number of struts 28 are provided to accommodate locking with pin-shape structures that permanently affix/connect the valve 10 material to the stent frame structure as further described in US Patent Application Serial No. 13/773,389 filed Feb. 21, 2013. A set of distal holes 12 at distal end 30 or "bottom" ventricular side of the stent advantageously provide attachment points (e.g., by suturing) of the valve leaflets to the stent frame as illustrated in Figs. 1A- 1F.

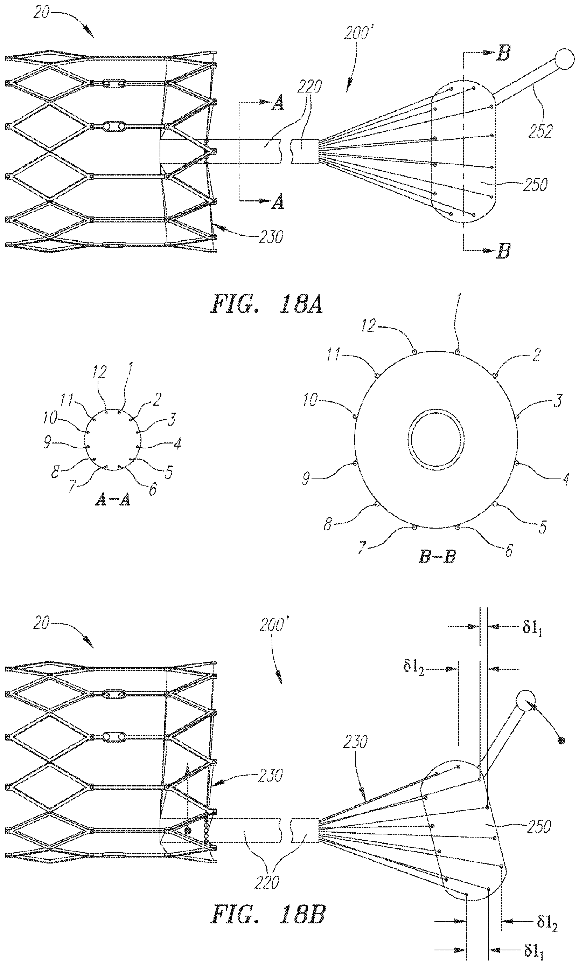

Figs. 18A and 18B are side views of the same stent frame 20 associated with a delivery system 200' related to that in Figs. 5A-10B, but including additional manipulation features. Specifically, delivery system 200' is adapted for controlling the lateral position of a heart valve device, for positioning or repositioning during deployment. Draw lines (or filaments) 230 (configured as in the referenced embodiments) are further connected to a pivot fitment 250 and a joystick-type handle 252.

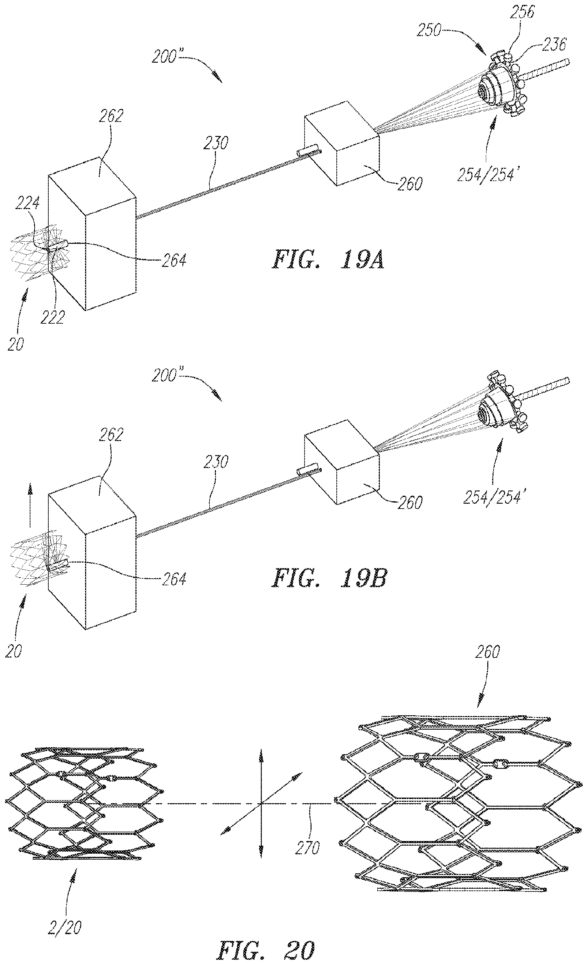

As shown in Figs. 19A and 19B loops or end ties 236 around spurs 256 may provide such a connection. As likewise shown, fitment 250 (altertatinvely, a boss, cap or housing) may ride upon or otherwise incorporate one or more spherical bearing surfaces 254/254'.

However configured, operation of sysem 200' is such that the angular ordering of the draw lines 230 in the overall heart valve (stent frame 20 shown) will correspond to the angular ordering of the draw lines on pivot fitment 250. Such activity is assured by the corresponding relationship of draw lines (or filaments) as shown in cross-sections A-A and B-B in Fig. 18A. The radial orientation of filaments 230 at the stent frame 20 and leading to the stent frame are matched with the radial orientation of the filaments at fitment 250 is indicated by the matching numeral position in the two cross-sectional views.

Therefore, as shown in Fig. 18B, tilting the pivot fitment 250 (e.g., by leaver arm/joystick 252) causes coordinated pull and release (or relaxation) of the draw lines proportional to the angular ordering and the direction of tilt to drive a corresponding change in the lateral position of the heart valve device (denoted by the directional arrows). Thus, the lateral position of the heart valve device can be controlled and manipulated by tilting the pivot fitment. While a joystick or similar interface can be incorporated into or connected to the pivot fitment to facilitate control of the tilt mechanism, other approaches including remote/robotic control are contemplated as well.

In any case, Figs. 19A and 19B are photographs of a functional prototype 200" of the delivery system embodiment diagrammatically shown in Figs. 18A and 18B. Here, blocks 260, 262 simulate the end contraint conditions of a catheter body. Between these, filaments 230 are visable (whereas they would generally be housed within a catheter body/sleeve). A short sleeve 264 extends from block 262 to simulate the distal portion of the catheter body 220 shown in Figs. 5A-10B, 18A and 18B including its side apertures 222 and an end hole 224.

In Fig. 19A, stent frame 20 and pivot fitment 250 are shown in a neutral or "home" position. While being tilted/turned, as shown in Fig. 19B, pivot fitment 250 reorents the filaments 230 to move stent 20 laterally in relation to sleeve 264.

Finally, Fig. 20 diagrammatically illustrates an alternative user interface for the Fig. 18A and 18B delivery system. Here, instead of using a handle, a model 260 of the implant 2 (or at least the stent frame 20) to be delivered is employed. The model may be a scale replica of the stent frame 20 and/or the entire implant 2. Generally, it will be configured in an expanded shape. However, it may be controlled so that its state of expansion matches that of implant 2. Alternatively, manipulation of the model expansion may alter the expansion state of the implant. Given all of these options, however, the model will generally at least serve as an interface for lateral valve positioning.

In which case, the model may be connected to the filaments in the same manner/fashion as the stent frame 20 to be manipulated along a catheter centerline 270 by movement of the model in any combination of lateral directions indicated by the axis arrows shown. Alternatively, model 260 may overlay and be connected to fitment 252 to which the filaments are connected (e.g., at spurs 254).

Use of the model 260 in manipulating the stent frame 20 and being able to visualize the direct correspondence of movement between the implant (via floroscopy or other medical imaging) to the sight of the model in hand may be particularly benefical to a physican in attempting ideal implant positioning and placement. In a method of use, the method may comprise at least partially deploying stent frame 20 by withdrawing a sheath 210 covering the stent frame and relaxing the filaments 230 passing through a catheter sleeve 220 and attached to the stent frame to expand the stent frame (e.g., as in such activity shown in Figs. 5A-5C). Then, a proximal interface such as a joystick or model is manipulated to move the stent frame laterally relative to the catheter sleeve by selectively tightening and relaxing the filaments (e.g., as in such activity shown in Fig. 18B relative to a zero or neutral position of fitment 252). Naturally, the device can be returned to center and then recompressed and/or resheathed for repositioning as well.

In the various delivery system architectures, the catheter/pusher shaft or sleeve may comprise a simple extrusion (e.g., PTFE, FEP, PEEK, PI etc.) or may be constructed using conventional catheter construction techniques and include a liner, braid support and outer jacket (not shown). Likewise, the various tubular members may comprise extrusion (per above), metal hypotube, etc. Further, the stent frame may be constructed using conventional laser cutting and electropolishing techniques and/or be otherwise constructed. In embodiments intended for tracking through a guide/delivery catheter without an incorporated sheath, a loading sheath (optionally peel-away or splittable) may be provided over the implant. Other typical percutaneous access instruments (such as wires, etc.), valves, and other hardware may also be employed in connection with the subject matter described herein.

The subject methods may include each of the physician activities associated with implant positioning, re-positioning, retrieval and/or release. Regarding these methods, including methods of manufacture and use, these may be carried out in any order of events which is logically possible, as well as any recited order of events.

Furthermore, where a range of values is provided, it is understood that every intervening value, between the upper and lower limit of that range and any other stated or intervening value in the stated range is encompassed within the invention.

Reference to a singular item includes the possibility that there are a plurality of the same items present. More specifically, as used herein and in the appended claims, the singular forms "a," "an," "said," and "the" include plural referents unless specifically stated otherwise. In other words, use of the singular forms allow for "at least one" of the subject item in the description above as well as the claims below. It is further noted that the claims may exclude any optional element and may explicitly limit each element to a "single" instance or "only one" such instance of that element. As such, this paragraph is intended to serve as antecedent basis for the use of such exclusive terminology as "solely," "only," "a single" and the like in connection with the recitation of claim elements, or the use of a negative limitation.

Without the use of such exclusive terminology, the terms "comprising," "including," and "having" in the claims shall allow for the inclusion of any additional element-irrespective of whether a given number of elements are enumerated in the claim, or the addition of a feature could be regarded as transforming the nature of an element set forth in the claims. Except as specifically defined herein, all technical and scientific terms used herein are to be given as broad a commonly understood meaning as possible while maintaining claim validity.

The breadth of the different embodiments or aspects described herein is not to be limited to the examples provided and/or the subject specification, but rather only by the scope of the issued claim language.

## Claims

1. A medical device delivery system (200) comprising:
an elongate sleeve (220) having a distal end (224) and a proximal end, wherein a plurality of apertures (222) are formed in the sleeve adjacent the distal end;
a plurality of filaments (230), each filament received within the sleeve from the proximal end, passing out and looping over the sleeve, and passing back into the sleeve through one of the plurality of apertures;
an elastic stent frame (20) comprising a plurality of struts (28), each strut including an aperture (22), and each filament passing through one of the strut apertures; wherein the elastic stent frame is self-expandable from a collapsed state to an expanded state; and
a handle (240) at the proximal end, the handle including a pivot fitment (250), the filaments connected to the pivot fitment spaced in a radial orientation matching a radial orientation of the filaments at the stent frame;
**characterised in that**
the pivot fitment is tiltable such that tilting of the pivot fitment causes selective tightening and relaxing of the filaments and a corresponding change in a lateral position of the elastic stent frame in the expanded state.

2. The delivery system of claim 1, further comprising a paired aperture (24) with each strut aperture, each paired aperture receiving a filament and orienting the filament along the strut.

3. The delivery system of claim 1 or claim 2, wherein the filaments pass out of the distal end of the sleeve.

4. The delivery system of any preceding claim, wherein the pivot fitment rides on a spherical bearing surface (254, 254').

5. The delivery system of any preceding claim, further comprising a joystick (252) to actuate the pivot fitment.

6. The delivery system of any one of claims 1 to 4, further comprising an implant model to actuate the pivot fitment, the model at least substantially resembling the stent frame in appearance.

7. The delivery system of claim 1, wherein the elastic stent frame is adapted to self-expand from a collapsed state to an expanded state, and wherein increased tension on the plurality of filaments causes the elastic stent frame to collapse from the expanded state.

8. The delivery system of claim 1, wherein the distal end (224) of the elongate sleeve (220) is within an inner space of the elastic stent frame.

## Patentansprüche

1. Ein Zuführsystem (200) für eine medizinische Vorrichtung, das Folgendes beinhaltet:
eine längliche Hülse (220) mit einem distalen Ende (224) und einem proximalen Ende, wobei eine Vielzahl von Öffnungen (222) in der Hülse benachbart zu dem distalen Ende gebildet ist;
eine Vielzahl von Fäden (230), wobei jeder Faden, der von dem proximalen Ende in der Hülse aufgenommen wird, aus der Hülse austritt und darüber eine Schleife bildet, und durch eine der Vielzahl von Öffnungen wieder in die Hülse eintritt;
einen elastischen Stentrahmen (20), beinhaltend eine Vielzahl von Streben (28), wobei jede Strebe eine Öffnung (22) umfasst und jeder Faden durch eine der Strebenöffnungen verläuft; wobei sich der elastische Stentrahmen von einem zusammengefalteten Zustand zu einem entfalteten Zustand selbstentfaltet; und
einen Griff (240) an dem proximalen Ende, wobei der Griff einen Schwenkanbau (250) umfasst, wobei die Fäden, die mit dem Schwenkanbau verbunden sind, in einer radialen Ausrichtung, die mit einer radialen Ausrichtung der Fäden an dem Stentrahmen übereinstimmt, beabstandet sind;
**dadurch gekennzeichnet, dass**
der Schwenkanbau derart kippbar ist, dass ein Kippen des Schwenkanbaus ein selektives Anspannen und Entspannen der Fäden und eine entsprechende Veränderung einer lateralen Position des elastischen Stentrahmens in dem entfalteten Zustand bewirkt.

2. Zuführsystem gemäß Anspruch 1, das ferner eine mit jeder Strebenöffnung gepaarte Öffnung (24) beinhaltet, wobei jede gepaarte Öffnung einen Faden aufnimmt und den Faden entlang der Strebe ausrichtet.

3. Zuführsystem gemäß Anspruch 1 oder Anspruch 2, wobei die Fäden aus dem distalen Ende der Hülse austreten.

4. Zuführsystem gemäß einem der vorhergehenden Ansprüche, wobei der Schwenkanbau auf einer sphärischen Lagerfläche (254, 254') gleitet.

5. Zuführsystem gemäß einem der vorhergehenden Ansprüche, das ferner einen Kreuzhebel (252) zum Betätigen des Schwenkanbaus beinhaltet.

6. Zuführsystem gemäß einem der Ansprüche 1 bis 4, das ferner ein Implantatmodell zum Betätigen des Schwenkanbaus beinhaltet, wobei das Modell dem Stentrahmen zumindest im Wesentlichen vom Aussehen her ähnelt.

7. Zuführsystem gemäß Anspruch 1, wobei der elastische Stentrahmen angepasst ist, um sich von einem zusammengefalteten Zustand zu einem entfalteten Zustand selbstentfaltet, und wobei eine erhöhte Spannung an der Vielzahl von Fäden bewirkt, dass sich der elastische Stentrahmen von dem entfalteten Zustand zusammenfaltet.

8. Zuführsystem gemäß Anspruch 1, wobei sich das distale Ende (224) der länglichen Hülse (220) in einem Innenraum des elastischen Stentrahmens befindet.

## Revendications

1. Un système de mise en place de dispositif médical (200) comprenant :
un manchon allongé (220) ayant une extrémité distale (224) et une extrémité proximale, une pluralité d'ouvertures (222) étant pratiquées dans le manchon dans le voisinage immédiat de l'extrémité distale ;
une pluralité de filaments (230), chaque filament étant reçu au sein du manchon depuis l'extrémité proximale, ressortant et faisant une boucle par-dessus le manchon, et repassant dans le manchon à travers l'une de la pluralité d'ouvertures ;
une ossature d'endoprothèse élastique (20) comprenant une pluralité d'entretoises (28), chaque entretoise incluant une ouverture (22), et chaque filament passant à travers l'une des ouvertures d'entretoise ; dans lequel l'ossature d'endoprothèse élastique est auto-expansible d'un état replié à un état déployé ; et
une poignée (240) au niveau de l'extrémité proximale, la poignée incluant un équipement à pivot (250), les filaments étant raccordés à l'équipement à pivot de façon espacée dans une orientation radiale concordant avec une orientation radiale des filaments au niveau de l'ossature d'endoprothèse ;
**caractérisé en ce que**
l'équipement à pivot est inclinable de sorte que le fait d'incliner l'équipement à pivot entraîne un serrage et un relâchement sélectifs des filaments et un changement correspondant dans une position latérale de l'ossature d'endoprothèse élastique dans l'état déployé.

2. Le système de mise en place de la revendication 1, comprenant en sus une ouverture appariée (24) avec chaque ouverture d'entretoise, chaque ouverture appariée recevant un filament et orientant le filament le long de l'entretoise.

3. Le système de mise en place de la revendication 1 ou de la revendication 2, dans lequel les filaments ressortent de l'extrémité distale du manchon.

4. Le système de mise en place de n'importe quelle revendication précédente, dans lequel l'équipement à pivot évolue sur une surface d'appui sphérique (254, 254').

5. Le système de mise en place de n'importe quelle revendication précédente, comprenant en sus un manche à balai (252) pour actionner l'équipement à pivot.

6. Le système de mise en place de l'une quelconque des revendications 1 à 4, comprenant en sus un modèle d'implant pour actionner l'équipement à pivot, le modèle ressemblant au moins substantiellement à l'ossature d'endoprothèse en apparence.

7. Le système de mise en place de la revendication 1, dans lequel l'ossature d'endoprothèse élastique est conçue pour une auto-expansion d'un état replié à un état déployé, et dans lequel une tension accrue exercée sur la pluralité de filaments entraîne le repliement de l'ossature d'endoprothèse élastique depuis l'état déployé.

8. Le système de mise en place de la revendication 1, dans lequel l'extrémité distale (224) du manchon allongé (220) se trouve au sein d'un espace interne de l'ossature d'endoprothèse élastique.
